(19) Europäisches Patentamt
European Patent Office
Office européen des brevets

(11) Veröffentlichungsnummer: **0 261 501 B1**

(12) **EUROPÄISCHE PATENTSCHRIFT**

(45) Veröffentlichungstag der Patentschrift: **31.03.93**

(51) Int. Cl.5: **C07C 43/12**, C08L 27/12

(21) Anmeldenummer: **87113156.1**

(22) Anmeldetag: **09.09.87**

Die Akte enthält technische Angaben, die nach dem Eingang der Anmeldung eingereicht wurden und die nicht in dieser Patentschrift enthalten sind.

# (54) Halogenhaltige Ether.

(30) Priorität: **17.09.86 DE 3631560**

(43) Veröffentlichungstag der Anmeldung:
**30.03.88 Patentblatt 88/13**

(45) Bekanntmachung des Hinweises auf die Patenterteilung:
**31.03.93 Patentblatt 93/13**

(84) Benannte Vertragsstaaten:
**BE DE FR GB IT NL**

(56) Entgegenhaltungen:
**EP-A- 0 090 498** **EP-A- 0 260 587**
**FR-A- 1 323 803** **FR-A- 1 373 014**
**FR-A- 2 306 189** **GB-A- 1 038 365**

**CHEMICAL ABSTRACTS, Band 99, Nr. 19, 7. November 1983, Seite 565, Zusammenfassung Nr. 157828t, Columbus, Ohio, US**

**BULLETIN OF THE ACADEMY OF SCIENCES OF THE USSR, DIVISION OF CHEMICAL SCIENCE, Band 33, Nr. 10, Teil 2, Okfober 1984, Seiten 2118-2120, Plenum Publishing Corp.; A.CH.-V. KIM et al.: "Preparation and properties of some derivatives of perfluoropropyl vinyl ether"**

(73) Patentinhaber: **HOECHST AKTIENGESELLSCHAFT**
**Postfach 80 03 20**
**W-6230 Frankfurt am Main 80(DE)**

(72) Erfinder: **Blickle, Peter, Dr.**
**Hattersheimer Strasse 14**
**W-6233 Kelkheim (Taunus)(DE)**
Erfinder: **Hintzer, Klaus, Dr.**
**Rupertusstrasse 3**
**W-8269 Burgkirchen(DE)**
Erfinder: **Löhr, Gernot, Dr.**
**Schneibsteinstrasse 6**
**W-8269 Burgkirchen(DE)**
Erfinder: **Schwertfeger, Werner, Dr.**
**Erlenstrasse 8**
**W-6306 Langgöns(DE)**

**EP 0 261 501 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Verbindungen der allgemeinen Formel (α)

$$X-CF_2-CF_2-O-\left[\overset{\displaystyle CF_3}{\overset{|}{C}}F-CF_2-O-\right]_{1-6}CFBr-CF_2Br \qquad (\alpha)$$

mit X = Br oder H sind in der EP-A-00 90 498 beschrieben. Sie werden für X = Br zum Beispiel durch Pyrolyse von Verbindungen der Formel

$$MOOC-CF_2-CF_2-O-\left[\overset{\displaystyle CF_3}{\overset{|}{C}}F-CF_2-O-\right]_{1-6}CFBr-CF_2Br$$

M = Alkali oder Erdalkalimetall

in Ausbeuten von maximal 33 % erhalten. Verbindungen mit X = H entstehen bei dieser Reaktion nur als Verunreinigungen, wenn nicht trocken gearbeitet wird.

Auch Verbindungen der allgemeinen Formel (β)

$CF_3$-$(CF_2)_n$-O-CFBr-$CF_2$Br (β),

mit n = 2 (Izv. Akad. Nauk SSSR, Ser.Khim 1984 2319, C.A. 102 131499s) oder n = 4 (DE-OS 2 614 333) gehören bereits zum Stand der Technik. Sie werden durch Bromierung der zugrundeliegenden Vinylether erhalten.

Bekannt sind schließlich auch Verbindungen der allgemeinen Formel (γ)

$$F-(CF_2)_3-\left[O-\overset{\displaystyle CF_3}{\overset{|}{C}}F-CF_2-\right]_n O-\overset{\displaystyle CF_3}{\overset{|}{C}}F-\overset{\displaystyle CF_3}{C}F-O-\left[CF_2-\overset{\displaystyle CF_3}{\overset{|}{C}}F-O-\right]_{n'}(CF_2)_3-F \qquad (\gamma),$$

die beispielsweise durch Kolbe-Elektrolyse der entsprechenden Carbonsäuren erhalten werden (Jpn, Kokai Tokkyo Koho JP 58 103 334, C.A. 99 157 828 t).

Die Erfindung betrifft Verbindungen der allgemeinen

Formel (I)

$$R_f-CFX-(CF_2)_n-\left[CF_2-O-\overset{\displaystyle CF_3}{\overset{|}{C}}F\right]_m-R_{f'} \qquad (I),$$

in der unabhängig voneinander sein können

- $R_f$ F oder Perfluoralkyl mit 1-3 C-Atomen, vorzugsweise F oder $CF_3$ und besonders bevorzugt F,
- X Wasserstoff, F, Br, Cl, vorzugsweise Wasserstoff, F, Br,
- n eine ganze Zahl von 1-10, vorzugsweise von 1-6, insbesondere von 1-5,
- m eine ganze Zahl von 0-5, vorzugsweise von 0-3, insbesondere von 0-2 und
- $R_{f'}$ -$CF_2$-O-CFBr-$CF_2$Br (a) oder

$$R_{f''}\text{-}CFX'\text{-}(CF_2)_{n'}\text{-}\left[CF_2\text{-}O\text{-}\overset{CF_3}{C}F\text{-}\right]_{m'} \qquad \text{(b)},$$

worin $R_{f''}$, X', n' und m' die gleiche Bedeutung haben wir $R_f$, X, n und m, mit der Maßgabe, daß im Falle von $R_{f'}$ gleich (a) und gleich F m mindestens 1, vorzugsweise 1, sein muß, und das im Falle von $R_{f'}$ gleich (b) X und X' nicht gleichzeitig gleich F sind und m + m' mindestens 1, vorzugsweise 2, 3 oder 4 sein muß.

Während beispielsweise Verbindungen wie

$$CF_3\text{-}CF_2\text{-}CF_2\text{-}O\text{-}\overset{CF_3}{C}F\text{-}\overset{CF_3}{C}F\text{-}O\text{-}CF_2\text{-}CF_2\text{-}CF_3,$$

also Vertreter der obigen allgemeinen Formel (γ), keine Löse-Eigenschaften für die in folgendem beschriebenen Fluorpolymeren aufweisen und mit Verbindungen wie $CF_3\text{-}CF_2\text{-}CF_2\text{-}O\text{-}CFBr\text{-}CF_2Br$ (Vertreter der allgemeinen Formel (β)) zwar Lösungen hergestellt werden können, die jedoch z.B. beim Gießen von Filmen nur unbefriedigende Filme ergeben, zeigen die erfindungsgemäßen Verbindugnen der allgemeinen Formel (I) gegenüber gleichen Fluorpolymeren überraschenderweise ein gutes Lösungsvermögen bzw. gute Resultate bei der Verarbeitung der Lösungen.

Als Beispiele für Verbindungen der obigen Formel (I) seien genannt:

$H\text{-}(CF_2)_3\text{-}O\text{-}CFBr\text{-}CF_2Br$

$H\text{-}(CF_2)_5\text{-}O\text{-}CFBr\text{-}CF_2Br$

$$H\text{-}(CF_2)_3\text{-}O\text{-}\overset{CF_3}{C}F\text{-}CF_2\text{-}O\text{-}CFBr\text{-}CF_2Br$$

$$CF_3\text{-}CHF\text{-}CF_2\text{-}CF_2\text{-}\left[O\text{-}\overset{CF_3}{C}F\text{-}CF_2\text{-}\right]_{0\text{-}2}O\text{-}CFBr\text{-}CF_2Br$$

$$CF_3\text{-}CF_2\text{-}CF_2\text{-}O\text{-}\overset{CF_3}{C}F\text{-}CF_2\text{-}O\text{-}CFBr\text{-}CF_2Br$$

$$Br\text{-}(CF_2)_3\text{-}\left[O\text{-}\overset{CF_3}{C}F\text{-}CF_2\text{-}\right]_{0,1}CFBr\text{-}CF_2Br$$

$$CF_3\text{-}CFBr\text{-}CF_2\text{-}\left[O\text{-}\overset{CF_3}{C}F\text{-}CF_2\text{-}\right]_{0,1}O\text{-}FBr\text{-}CF_2Br$$

$$H\text{-}(CF_2)_3\text{-}\left[O\text{-}\overset{CF_3}{C}F\text{-}CF_2\text{-}\right]_{0,1}O\text{-}\overset{CF_3}{C}F\text{-}\overset{CF_3}{C}F\text{-}O\text{-}\left[CF_2\text{-}\overset{CF_3}{C}F\text{-}O\text{-}\right]_{0,1}(CF_2)_3\text{-}H$$

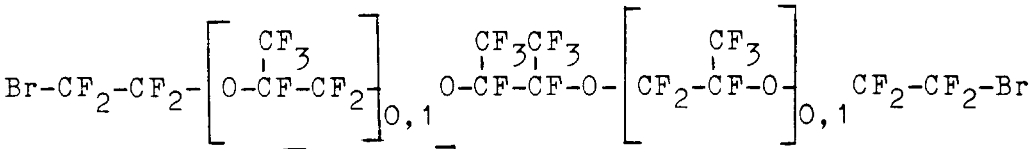

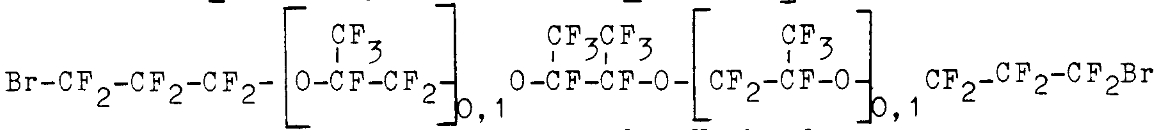

Die Herstellung der erfindungsgemäßen Verbindungen erfolgt analog bekannten Sytheseverfahren. Verbindungen der allgemeinen Formel (I), in denen $R_f'$ für $CF_2$-O-CFB-$CF_2$Br steht, können beispielsweise erhalten werden durch Addition von Brom an ungesättigte Verbindungen der allgemeinen

Formel (II)

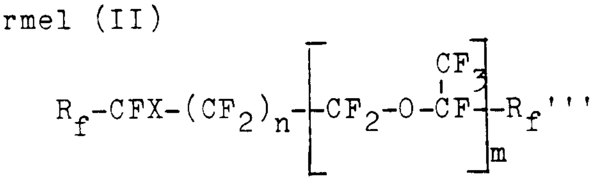

(II),

in der $R_f$, X, n und m die obengenannten Bedeutungen haben und $R_f'''$ -$CF_2$-O-CF = $CF_2$ ist.

Die Verbindungen der allgemeinen Formel II ($R_{f'''}$ = -$CF_2$-O-CF = $CF_2$) sind z.B. durch die nachstehende Reaktionsfolge leicht herstellbar:

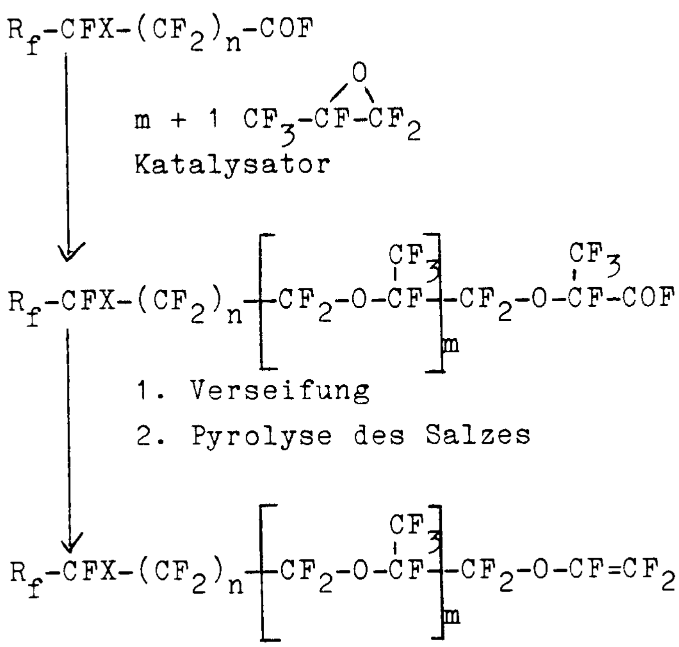


(III)

(IV)

(V)

Bezüglich näherer Einzelheiten sei auf Angew. Chem. 97, 164 (1985) sowie auf R.E. Banks, Preparation, Properties, and Ind. Appl. of Organofluorine Compound Ellis Horwood Ltd. 1982, Seite 257 verwiesen.

Durch Kolbe-Elektrolyse können Verbindungen der allgemeinen

Formel (I) mit 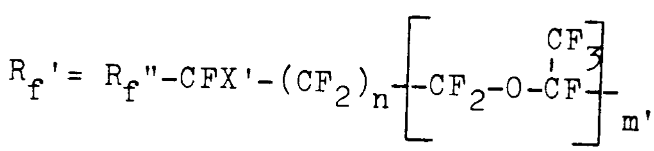

erhalten werden. Beim Einsatz von zwei verschiedenen Carbonsäuren können dabei auch Kolbe-Produkte hergestellt werden, die nicht symmetrisch aufgebaut sind:

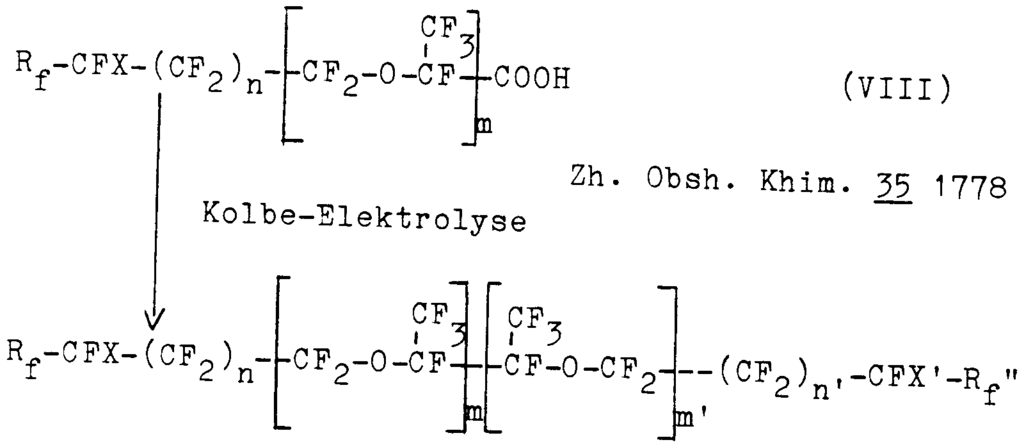


(VIII)

Zh. Obsh. Khim. 35 1778 (1965)

(IX)

Die erfindungsgemäßen Verbindungen können als Zwischenprodukte sowie als wärmeübertragende Flüssigkeiten eingesetzt werden. Vorzugsweise finden sie jedoch als Lösungsmittel für Fluorpolymere Verwendung, beispielsweise solche, wie sie in der DE-OS 2 905 457 und der DE-OS 3 036 410, in der EP-A-00 66 369 und der EP-A-00 88 285 sowie in der WO 81/01158 beschrieben sind.

Bevorzugt enthalten diese Fluorpolymere die Gruppen

$-SO_2F$ (A)

und/oder

-CG (G = Stickstoff, -OOR oder $-ON(R)_2$, wobei R $C_1$-$C_{10}$-Alkyl, Aryl, Aralkyl, vorzugsweise $C_1$-$C_8$-Alkyl darstellt) (B)

wobei die Gruppen (B) bevorzugt sind. Vorzugsweise bedeutet die Gruppe (B) -COOR.

Besonders bevorzugt sind hier Fluorpolymere zu nennen, welche die wiederkehrenden Einheiten mit der allgemeinen Formel (C) entweder allein oder zusammen mit anderen wiederkehrenden Einheiten enthalten:

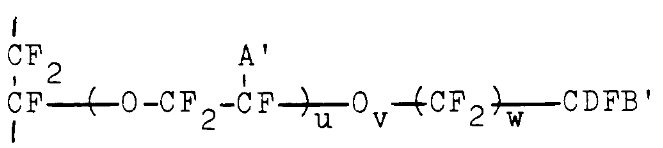
(C)

worin

| | |
|---|---|
| u = | 0, 1 oder 2, vorzugsweise 0 oder 1, |
| v = | 0 oder 1, vorzugsweise 1, |
| w = | 1 - 7, vorzugsweise 2 - 5, |
| A' und B' = | unabhängig voneinander F oder $CF_3$, vorzugweise A' = $CF_3$ und B' = F und |
| D = | Wasserstoff, Cl, Br, CG (G die gleiche Bedeutung wie oben) und weniger bevorzugt $SO_2F$ bedeuten. |

Diese wiederkehrenden Einheiten (C) können ggf. in Mischung untereinander das Fluorpolymere aufbauen. Vorzugsweise finden sich in diesem jedoch zusätzlich noch wiederkehrende Einheiten der allgemeinen Formel (D)

$-CF_2-CFE-$ (D)

worin E für Cl, F, $R_f$ oder $OR_f$ ($R_f$ = $CF_3$ oder ggf. durch O-Atome unterbrochener $C_2$-$C_8$-Perfluoralkylrest) steht. Bevorzugt bedeutet E = F, $CF_3$, $-O-CF_2-CF_2-CF_3$ oder

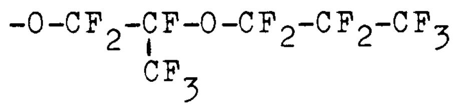

und insbesondere F.

Auch hier können diese wiederkehrenden Einheiten ggf. in Mischung vorliegen. Bevorzugt im Rahmen dieser Fluorpolymeren sind solche, welche aus der wiederkehrenden Einheit (C)

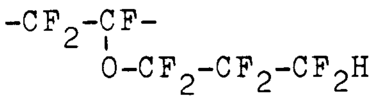

oder

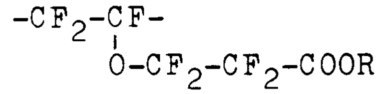

und aus der wiederkehrenden Einheit (D) $-CF_2-CF_2-$ im Falle von Bipolymeren bzw. $-CF_2-CF_2-$ und -CF-CFE- (E = obige Bedeutung mit Ausnahme von F) im Falle von Terpolymeren bestehen. Der Gehalt an (C) beträgt im Falle von Bi- bzw. Terpolymeren 10 bis 50 Mol-%, vorzugsweise 13 bis 45 Mol-%, bezogen auf das Gesamtpolymere.

Beispiele für zu den wiederkehrenden Einheiten (C) führenden Monomeren sind: $CF_2 = CFOCF_2CF_2SO_2F$,

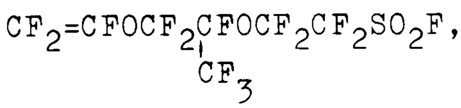

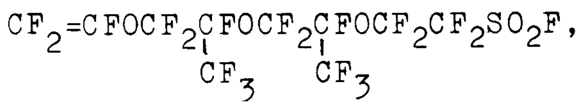

$CF_2 = CFCF_2CF_2SO_2F$,
$CF_2 = CFO(CF_2)_{1-6}COOCH_3$, $CF_2 = CF(CF_2)_{0-8}COOCH_3$

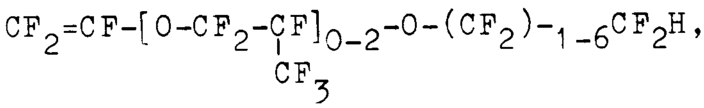

$CF_2 = CFOCF_2CF(CF_3)OCF_2CF_2COOCH_3$,
$CF_2 = CF\text{-}O\text{-}CF_2CF(CF_3)O(CF_2)_3COOCH_3$,
$CF_2 = CF\text{-}O\text{-}CF_2CF(CF_3)OCF_2COOCH_3$.

Im Zusammenhang mit der wiederkehrenden Einheit (D) sind als Beispiele Tetrafluorethylen, Trifluorchlorethylen, Hexafluorpropen, Perfluor(propylvinylether) und Perfluorpropoxy(propylvinylether) zu nennen.

Die Herstellung derartiger aus (C) und ggf. (D) bestehender Fluorpolymerer ist beispielsweise in der EP-A-00 88 285 und der EP-A-0 171 696 beschrieben.

Das Molekulargewicht (Zahlenmittel) der Fluorpolymeren liegt im allgemeinen zwischen 50000 und 1000000, vorzugsweise zwischen 100000 und 500000. Das Äquivalentgewicht (bei Vorhandensein der Gruppen (A) und/oder (B)) beträgt 400 bis 1800, vorzugsweise 450 bis 1500.

Die Herstellung der Lösungen der Fluorpolymeren kann bei Temperaturen von 20 °C bis zum Siedepunkt des entsprechenden Lösungsmittels, vorzugsweise bei Temperaturen von 50 °C bis 10 °C unterhalb des Siedepunktes erfolgen. Gegebenenfalls kann dabei auch unter überatmosphärischem Druck gearbeitet

werden, um höhere Temperaturen zu erzielen. Im allgemeinen ist jedoch Normaldruck bevorzugt. Es ist von Vorteil, den Lösevorgang durch Rühren udgl. der Lösung zu unterstützen und das Fluorpolymere in möglichst feinverteilter Form einzusetzen. Die Konzentration (der Feststoffgehalt) der erhaltenen Lösungen liegt üblicherweise zwischen 0,5 und 30 Gew.-%, vorzugsweise zwischen 1 und 25 Gew.-%.

Die so erhaltenen Lösungen zeichnen sich vor allem durch ihre Stabilität aufgrund des Fehlens von funktionellen Gruppen im Lösungsmittel, der Fähigkeit des Lösungsmittels, Polymere mit oder ohne polare Gruppen gleichermaßen gut zu lösen, ihrem hohen Feststoffgehalt wegen der guten Löse-Eigenschaften der Lösungsmittel und des Umstandes aus, daß daraus Formkörper (Membranen) ohne größere thermische Belastung des Fluorpolymeren hergestellt werden können.

Die Lösungen können auf den verschiedensten Gebieten eingestzt werden, wie sie beispielsweise in der DE-OS 2.905.467 und der DE-OS 3.036.410 sowie der EP-A-00 66 369 beschrieben sind. Bevorzugt werden sie zur Herstellung oder Repartur von Membranen insbesondere für Flüssigkeitsdurchdringungsprozesse benutzt, wie sie bei der Ultra- bzw. Hyperfiltration, Dialyse (umgekehrte Osmose) Elektrolyse, insbesondere von wäßrigen Kochsalzlösungen, sowie in Brennstoffzellen auftreten. Als weitere Einsatzgebiete kommen Membranen für Ionenaustauschprozesse, für Massentransporte sowie für katalytische Zwecke in Betracht.

Beispiele

I. Herstellung von Verbindungen mit $CF_2$-O-CFBr-$CF_2$Br-Gruppen

a) Herstellung der Vinylether

Allgemeine Arbeitsvorschrift:

In einem Glaskolben oder Glasautoklaven, der mit einem Flügelrührer ausgestattet ist, wurde das betreffende Carbonsäurefluorid und der Katalysator vorgelegt. Unter intensivem Rühren wurde dann Hexafluorpropenepoxid aufgedrückt, wobei das Gas unter exothermer Reaktion aufgenommen wurde. Durch Kühlung wurde die Innentemperatur bei 20 - 40 °C gehalten. Nach dem Ende der Reaktion wurde das Gemisch destilliert. Die Reaktion verlief nach der Gleichung:

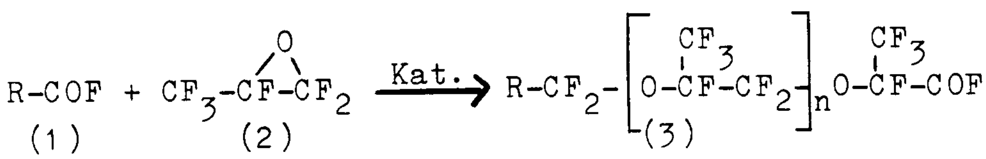

(1) (2) (3)

| R = | Mol (1) | Mol (2) | Katalysator | Ausbeute (3) | kg |
|---|---|---|---|---|---|
| $H-CF_2-CF_2$ | 6.28 | 12.05 | CsF (0.2 Mol), Tetraglyme* (100 ml) | (n = 0) 57 %<br>(n = 1) 16 % | 73°C<br>127°C |
| $H-(CF_2)_4$ | 1.8 | 2.3 | CsF (0.1 Mol), Tetraglyme (50 ml) | (n = 0) 76 % | 116 - 118°C |
| $Br-CF_2-CF_2$ | 1.54 | 1.8 | CsF (0.1 Mol), Tetraglyme (50 ml) | (n = 0) 61 %<br>(n = 1) 3 % | 98 - 99°C<br>147 - 148°C |

* Diethylenglykoltetramethylether

Die Säurefluoride der Formel (3) wurden mit wäßriger Kalilauge unter Eiskühlung verseift und die neutrale Salzlösung im Vakuum eingedampft. Das trockene Salz wurde bei 200 - 250 °C pyrolysiert, und der entstehende Vinylether in einer kältefalle (-78 °C) kondensiert. Die Destillation ergab die Vinylether (4) mit Ausbeuten größer als 80 %.

$$(3) \xrightarrow{KOH/H_2O} \text{Salz} \xrightarrow{\text{Pyrolyse}} R-CF_2-\left[O-\underset{}{\overset{CF_3}{C}}F-CF_2-\right]_n O-CF=CF_2$$

(4)

| (4) R | (n) | Siedepunkt |
|---|---|---|
| $H-(CF_2)_2$ | (0) | 55 - 56°C |
| | (1) | 112 - 114°C |
| $H-(CF_2)_4$ | (0) | 102°C |
| $Br-(CF_2)_2$ | (0) | 82°C |
| | (1) | 138°C |

b) Anlagerung von Brom an Vinylether

In einem Glaskolben, der mit einem Magnetrührer, Rückflußkühler und Tropftrichter ausgestattet war, wurde der Vinylether vorgelegt. Unter Belichtung mit einer Tageslichtlampe tropfte man so lange Brom zu, bis keine Entfärbung mehr auftrat. Die Innentemperatur wurde durch Kühlung mit Eis zwischen 20 und 100 °C gehalten. Die Aufarbeitung des Ansatzes erfolgte entweder durch Waschen mit Natronlauge und

Wasser und anschließende Trocknung über $P_2O_5$ mit nachfolgender Destillation oder durch direkte Destillation des Reaktionsgemisches.

| "Dibromide" | | Kp.: °C/mbar | Ausbeute % |
|---|---|---|---|
| $H-(CF_2)_3-O-\left[\overset{CF_3}{\overset{\mid}{CF}}-CF_2-O-\right]_n-CFBr-CF_2Br$ | | | |
| | n=0 | 58-59°/80 | 92 |
| | n=1 | 83-84°/40 | 83 |
| $H-(CF_2)_5-O-CFBr-CF_2Br$ | | 68°/27 | 92 |
| $CF_3-(CF_2)_2-O-\underset{CF_3}{CF}-CF_2-O-\underset{CF_2Br}{CFBr}$ | | 160/1013 | 85 |
| $Br-(CF_2)_3-O-\overset{CF_3}{\overset{\mid}{CF}}-CF_2-O-CFBr-CF_2Br$ | | 114°/67 | 79 |
| $CF_3-CHF-(CF_2)_2-O-\left[\overset{CF_3}{\overset{\mid}{CF}}-CF_2-O-\right]_m-CFBr-CF_2Br$ | | | |
| | m=0 | 66-67°/64 | 82 |
| | m=1 | 101°/67 | 77 |
| | m=2 | 83-84°/6.7 | 80 |

II Herstellung der Kolbe-Produkte

Die Kolbe-Produkte der allgemeinen Formel

$$I\ (R' = \left[\overset{CF_3}{\overset{\mid}{CF}}-O-CF_2-\right]_{m'}(CF_2)_{n'}-CFX'-R_{f''})$$

wurden durch Kolbe-Elektrolyse der entsprechenden Carbonsäuren erhalten.

a) In einer Glastopfelektrolysezelle mit Platinanode und kathode (je 50 $cm^2$) wurden 331 g

$$H-(CF_2)_2-(CF_2-O-\underset{CF_3}{\underset{\mid}{CF}})_2-COOH$$

in eine Mischung aus 400 ml Methanol, 200 ml Wasser und 1,5 g NaOH elektrolysiert. Nach 145 % der theoretisch erforderlichen Strommenge wurde das erhaltene Produkt abgetrennt, mit $NaHCO_3$-Lösung sowie Wasser gewaschen und dann über $Na_2SO_4$ getrocknet. Die fraktionierte Destillation ergab 242 g (81 %)

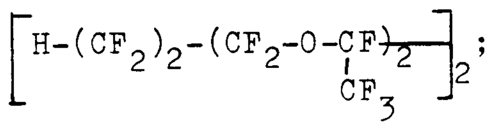

Kp. 70 °C/0,75 mbar

b) Aus

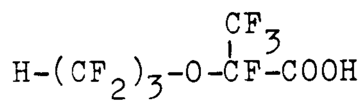

wurde unter entsprechenden Bedingungen der Ether

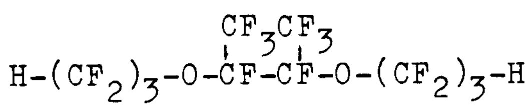

hergestellt (Kp. 103 - 104 °C/233 mbar; Ausbeute 67 %).

c) In der gleichen Elektrolysezelle wie in Beispiel II a) wurde ein Gemisch von 108 g

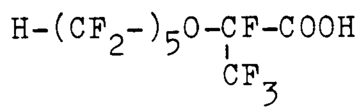

und 38,3 g $H\text{-}CF_2\text{-}CF_2COOH$ in einer Lösung von je 120 ml Methanol und Wasser mit 0,5 g NaOH elektrolysiert. Nach 160 % der theoretische erforderlichen Strommenge wurde das Produktgemisch abgetrennt, mit Natronlauge und Wasser gewaschen und dann fraktioniert destilliert. Die höhersiedenden Fraktionen ergaben:

32 g 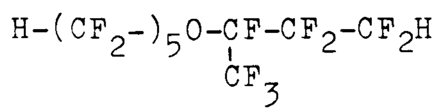

(Sdp. 56 °C/40 mbar) und

43,6 g 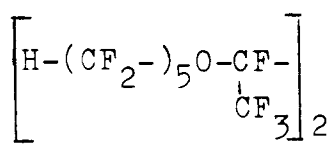

(Sdp. 90 °C/10 mbar)

d) Ebenso wurde ein Gemisch aus 97,8 g

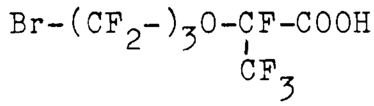

und 38 g $H-CF_2-CF_2-COOH$ elektrolysiert.
Nach Aufarbeitung und Destillation wurden erhalten:

43,7 g 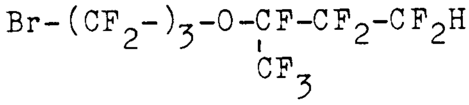

(Sdp. 127° C) und

48,3 g 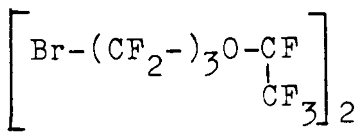

(Sdp. 160° C/330 mbar)

III Anwendungsbeispiele:

1) 10 g eines Fluorpolymeren aus Tetrafluorethylen (TFE) und ω-Hydroperfluor-(propylvinylether) (HPPVE), das gemäß Beispiel 1 der EP-A-0 171 696 hergestellt worden war und 21,5 Mol-% HPPVE enthielt, wurden in 90 g des Lösungsmittels mit der Formel

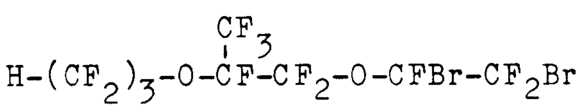

(Kp. 83 - 84° C/40 mbar) bei 140° C 3 Stunden lang gerührt. Es entstand eine völlig klare Lösung, woraus sich z.B. schöne klare Folien herstellen ließen.
2) Das Fluorpolymere gemäß Beispiel 1 wurde nach dem in Beispiel 1 der EP-A 00 88 285 beschriebenen Verfahren in die Methylesterform überführt. 10 g eines solchen Fluorpolymeren wurden danach 5 Stunden bei 150° C in 80 g des in Beispiel 1 verwendeten Lösungsmittels gerührt. Man erhielt eine völlig klare Lösung mit einem Feststoffgehalt von 11,1 Gew.-%. Aus einer solchen Lösung wurden Filme hergestellt, die nach der Hydrolyse mit NaOH z.B. als Ionenaustauschermembranen bei der Chloralkali-Elektrolyse Verwendung finden. Wurde eine solche Membran in eine Elektrolyse-Zelle, die mit einer Titan-Streckmetallanode und einer V4A-Kathode versehen war, eingebaut, so erhielt man bei einer Stromdichte von 3000 $A/m^2$, einer Anolytkonzentration von 205 g $NaCl/1H_2O$ und einer Laugenlkonzentration von 36 % NaOH eine Stromausbeute von 94 % und eine Zellspannung von 3,45 V.
3) Zu 5 g des Copolymeren aus Beispiel 2 wurden 50 g des Lösungsmittels der Formel

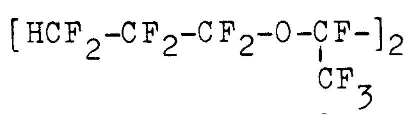

(Kp.: 150° C) gegeben. Es wurde 2 Stunden bei 100° C gerührt und eine klare Lösung des Polymeren enhalten.

4) (Vergleich): Zu 5 g des Coppolymeren aus Beispiel 2 wurden 150 g des Lösungsmittels der Formel

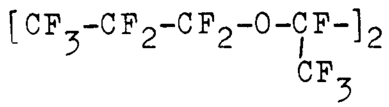

(KP.: 134 °C) gegeben. Es wurde 7 Stunden bei 120 °C gerührt; das Polymere war lediglich stark gequollen, aber nicht gelöst.

5) (Vergleich): Zu 10 g des Copolymeren aus Beispiel 1 wurden 200 g $CF_3$-$CF_2$-$CF_2$-O-CFBr-$CF_2$Br (Kp. 112 °C) gegeben. Man rührte 7 Stunden bei 110 °C. Die aus der erhaltenen Lösung hergestellten Folien waren im Gegensatz zu denen aus Beispiel 1 trübe und wiesen eine sehr geringe mechanische Festigkeit auf.

6) Zu je 5 g eines Terpolymeren aus TFE, Perfluorpropylvinylether (PPVE) und HPPVE wurden 70 g H-$(CF_2)_3$-O-CF($CF_3$)-O-$(CF_2)_3$-H (Ansatz I) bzw. 100 g $CF_3$-$CF_2$-$CF_2$-O-CFBr-$CF_2$Br (Ansatz II-Vergleich) gegeben. Ansatz I wurde 3 Stunden bei 95 °C gerührt. Ansatz II wurde 3 Stunden bei 105 °C gerührt. Die beiden erhaltenen Lösungen wurden zu Folien vergossen: Ansatz I ergab schöne, klare und mechanisch stabile Folien; die Folien aus Ansatz II waren sehr trübe und wiesen ungenügende mechanische Festigkeit auf.

## Patentansprüche

1. Verbindungen der allgemeinen Formel (I)

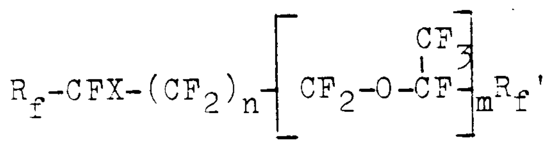

(I),

in der

$R_f$ F oder Perfluoralkyl mit 1-3 C-Atomen,
X Wasserstoff, F, Br, Cl,
n eine ganze Zahl von 1 - 10,
m eine ganze Zahl von 0 - 5 und
$R_f'$ -$CF_2$-O-CFBr-$CF_2$Br (a) oder

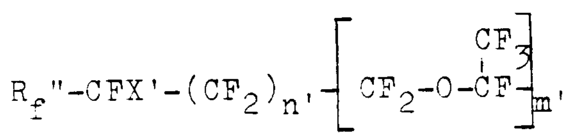

(b) darstellen,

worin $R_f''$, X', n' und m' die gleiche Bedeutung haben wie $R_f$, X, n und m mit der Maßgabe, daß im Falle von $R_f'$ gleich (a) und X gleich F m mindestens 1 sein muß, und daß im Falle von $R_f'$ gleich (b) X und X' nicht gleichzeitig gleich F sind und m + m' mindestens 1 sein muß.

2. Verbindung nach Anspruch 1, dadurch gekennzeichnet, daß $R_f$ für F oder $CF_3$ steht.

3. Verbindung nach Anspruch 1 und/oder 2, dadurch gekennzeichnet, daß X gleich Wasserstoff, F oder Br ist.

4. Verbindung nach mindestens einem der Ansprüche 1 bis 3, dadurch gekennzeichnet, daß n eine ganze Zahl von 1 bis 6 ist.

5. Verbindungen nach mindestens einem der Ansprüche 1 bis 4, dadurch gekennzeichnet, daß m eine ganze Zahl von 0 bis 3 ist.

## Claims

1. A compound of the formula (I)

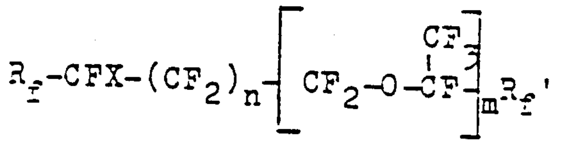 (I)

in which
$R_f$ denotes F or perfluoroalkyl having 1-3 carbon atoms,
X denotes hydrogen, F, Br or Cl,
n denotes an integer from 1-10,
m denotes an integer from 0-5, and
$R_f'$ denotes $-CF_2-O-CFBr-CF_2Br$ (a) or

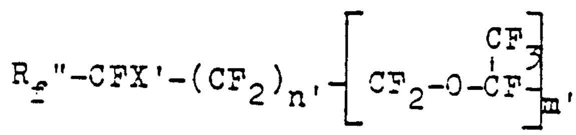 (b)

in which $R_f''$, X', n' and m' have the same meaning as $R_f$, X, n and m, with the proviso that, where $R_f'$ equals (a) and X equals F, m must be at least 1, and that, where $R_f'$ equals (b), X and X' are not simultaneously equal to F and m + m' must be at least 1.

2. A compound as claimed in claim 1, wherein $R_f$ represents F or $CF_3$.

3. A compound as claimed in claim 1 and/or 2, wherein X is hydrogen, F or Br.

4. A compound as claimed in at least one of claims 1 to 3, wherein n is an integer from 1 to 6.

5. A compound as claimed in at least one of claims 1 to 4, wherein m is an integer from 0 to 3.

## Revendications

1. Composés de formule générale (I)

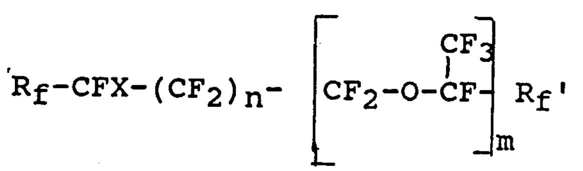 (I),

dans laquelle,
$R_f$ représente F ou un groupe perfluoralkyle ayant 1 à 3 atomes de carbone,
X représente un atome d'hydrogène, F, Br, Cl;
n est un nombre entier valant 1 à 10;
m est un nombre entier valant 0 à 5, et
$R_f'$ représente $-CF_2-O-CFBr-CF_2Br$ (a) ou

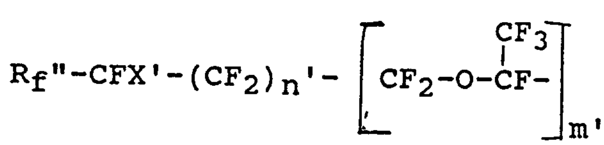 (b),

formule dans laquelle $R_f''$, X', n' et m' ont le même sens que $R_f$, X, n et m, avec la réserve que, au cas où $R_f'$ représente (a) et X représente F, le nombre m doit valoir au moins 1 et que, au cas où $R_f'$ représente (b), les symboles et X' ne peuvent pas simultanément représenter chacun F, et la somme (m + m') doit valoir au moins 1.

**2.** Composés selon la revendication 1, caractérisés en ce que $R_f$ représente F ou $CF_3$.

**3.** Composés selon la revendication 1 ou 2, caractérisé en ce que X représente un atome d'hydrogène, F ou Br.

**4.** Composés selon l'une au moins des revendications 1 à 3, caractérisés en ce que n est un nombre entier valant 1 à 6.

**5.** Composés selon l'une au moins des revendications 1 à 4, caractérisés en ce que m est un nombre entier valant 0 à 3.